# DEMANDE DE BREVET EUROPEEN

(11) **EP 2 158 941 A2**
(43) Date de publication de la demande: **03.03.2010**
(21) Numéro de dépôt: 09157475.6
(22) Date de dépôt: 07.04.2009
(51) Int. Cl.: A61Q 3/02, A61K 8/06, A61K 8/81, A61K 8/73

(54) **Vernis à ongles sous forme d'émulsion, comprenant un agent gélifiant particulier**

(30) Priorité: 18.04.2008 FR 0852654
(71) Demandeur: L'Oréal, 75008 Paris (FR)
(72) Inventeur: Rolland, Nicolas, 92600, Asnieres sur Seine (FR); Guerchet, Laurence, 94550, Chevilly La Rue (FR)
(74) Mandataire: Casalonga, Axel

(57) **Abrégé**

L'invention concerne une composition de vernis à ongle sous forme d'émulsion, comprenant un ou plusieurs polymères hydrosolubles ou hydrodispersibles obtenus à partir d'acide acrylamido-2-méthylpropane-sulfonique (ou AMPS) ou d'un de ses sels et de monomères hydrosolubles à insaturation(s) éthylénique(s) comme agent gélifiant Elle concerne également un procédé de maquillage mettant en oeuvre ladite composition.

## Description

La présente invention concerne une composition de vernis à ongle aqueuse sous forme d'émulsion, comprenant au moins un agent gélifiant tel que défini ci-dessous, et un procédé de maquillage des ongles mettant en oeuvre ladite composition.

Généralement, le maquillage des ongles ou des faux ongles est réalisé à l'aide de compositions liquides de maquillage, encore communément appelées vernis à ongles. Ce vernis à ongles est appliqué généralement sous la forme de couches superposées à la surface de l'ongle à maquiller, en respectant une étape de séchage intermédiaire entre chaque couche de vernis appliqué. En fait, ce mode de maquillage ne s'avère pas totalement satisfaisant.

Tout d'abord, son application nécessite un certain temps. D'autre part, ce type de maquillage implique d'être répété à bref délai compte tenu de sa tenue insuffisante. En effet, très rapidement, généralement au terme de trois à cinq jours, le vernis appliqué s'écaille et sa brillance diminue. Il est alors nécessaire de procéder à une étape de démaquillage et une nouvelle opération de maquillage.

Par ailleurs, les vernis à ongles classiques impliquent généralement la mise en oeuvre de solvants volatils qui génèrent, lors de l'application, une odeur inconfortable.

Plusieurs alternatives ont déjà été proposées pour tenter de surmonter, au moins en partie, les inconvénients précités. Ainsi, des produits de maquillage des ongles ont été proposés sous la forme de kit de deux compositions liquides de vernis à ongles. Toutefois, l'amélioration de la tenue est dans ce cas acquise au détriment des conditions d'application qui multiplient par deux le nombre de couches à appliquer.

Une autre alternative a consisté à développer des compositions de vernis à ongles à base de dispersion de polymères filmogènes en phase aqueuse, et donc satisfaisantes en terme olfactif. Malheureusement, les vernis correspondants s'avèrent présenter une tenue insuffisante dans le temps.

Enfin, les vernis à ongles classiques sont en grande majorité anhydres, et ne permettent donc pas l'utilisation de composés hydrosolubles comme des actifs hydrosolubles.

La demanderesse a trouvé qu'en préparant un vernis à ongles sous la forme d'une émulsion comprenant au moins un agent gélifiant particulier tel que décrit ci-dessous, on pouvait obtenir une application aisée et rapide, la formation d'un film sec homogène et brillant, adhérent et flexible, et une teneur réduite en solvant(s) organique(s).

Cette émulsion présente également l'avantage d'être stable et fluide.

La présente invention a donc pour objet une composition de vernis à ongle sous forme d'émulsion, comprenant un ou plusieurs agents gélifiants tels que définis ci-dessous.

Elle a encore pour objet un procédé de maquillage des ongles mettant en oeuvre ladite composition.

D'autres objets et caractéristiques, aspects et avantages de l'invention apparaîtront encore plus clairement à la lecture de la description et de l'exemple qui suit.

La composition de vernis à ongle sous forme d'émulsion comprend un ou plusieurs polymères hydrosolubles ou hydrodispersibles obtenus à partir d'acide acrylamido-2-méthylpropane-sulfonique (ou AMPS) ou d'un de ses sels et de monomères hydrosolubles à insaturation(s) éthylénique(s) comme agent(s) gélifiant(s).

Par « vernis à ongle », on entend une composition susceptible de former un film sur l'ongle.

La composition selon l'invention se présente sous forme d'émulsion ; elle comprend donc au moins une phase continue et au moins une phase dispersée.

De préférence, la phase continue comprend majoritairement une ou plusieurs huiles (on parle alors de phase continue huileuse) ou un ou plusieurs solvants organiques (on parle alors de phase continue organique), et la phase dispersée est aqueuse.

La phase continue comprend de préférence majoritairement une ou plusieurs huiles ou un ou plusieurs solvants organiques, c'est-à-dire une quantité d'huile(s) et/ou de solvant(s) organique(s) allant de préférence de 40 à 99,99 % en poids, mieux encore de 60 à 99,99 % en poids par rapport au poids total de la phase continue.

En particulier, la phase continue comprend au moins un solvant organique et la composition se présente sous forme d'émulsion eau-dans-solvant organique. Une telle émulsion permet, par la présence d'une phase aqueuse dispersée, l'incorporation d'actifs hydrosolubles.

De préférence, la composition comprend une quantité allant de 10 à 85 % en poids, mieux de 30 à 80 % en poids de phase continue par rapport au poids total de la composition. De préférence, la composition comprend une quantité allant de 15 à 90 % en poids, mieux de 20 à 70 % en poids de phase dispersée par rapport au poids total de la composition.

De préférence, la phase continue et la phase dispersée de l'émulsion sont présentes en un rapport pondéral allant de 1/10 à 6, mieux de 2/5 à 4.

A titre d'exemples d'agent gélifiant utilisable dans la composition selon l'invention, on peut notamment citer :
- les copolymères obtenus à partir d'AMPS ou d'un de ses sels et d'acrylamide ou de méthylacrylamide, comme, par exemple, le copolymère réticulé acrylamide/acrylamido-2-méthylpropane-sulfonate de sodium en émulsion dans l'isoparaffine en C₁₃-C₁₄ et le laureth-7 (nom CTFA : polyacrylamide/C₁₃-C₁₄ Isoparaffin/laureth-7) commercialisé sous la dénomination SEPIGEL 305 par la société SEPPIC, ou le copolymère réticulé acrylamide/acrylamido-2-méthylpropane-sulfonate de sodium en émulsion inverse à 40 % dans l'isohexadécane et le polysorbate-80 (nom CTFA : Acrylamide/Sodium Acryloyldimethyltaurate/ Isohexadecane/polysorbate-80) commercialisé sous la dénomination SIMULGEL 600 par la société SEPPIC ;
- les copolymères d'AMPS ou d'un de ses sels et de vinylpyrrolidone ou de vinylformamide, tels que les produits Ammonium Acryloyldimethyltaurate/VP Copolymer (nom INCI) commercialisés sous la dénomination ARISTOFLEX AVC par la société CLARIANT ;
- les copolymères d'AMPS ou d'un de ses sels et d'acrylate de sodium, comme, par exemple, le copolymère réticulé AMPS/acrylate de sodium en émulsion inverse dans un mélange eau/isohexadécane/oléate de sorbitane (nom CTFA : Acrylamide/Sodium Acryloyldimethyltaurate/Isohexadecane/polysorbate-80) commercialisé sous la dénomination SIMULGEL EG par la Société SEPPIC ; et
- les copolymères d'AMPS ou d'un de ses sels et d'acrylate d'hydroxyéthyle, comme, par exemple, le copolymère réticulé AMPS/ acrylate d'hydroxyéthyle en émulsion inverse dans un mélange polysorbate 60/squalane (commercialisé sous la dénomination SIMULGEL NS par la société SEPPIC).

Plus particulièrement, l'agent gélifiant est choisi parmi les copolymères obtenus à partir d'AMPS ou d'un de ses sels et d'acrylamide ou de méthylacrylamide.

Le ou les agents gélifiants sont présents en une quantité allant de préférence de 0,01 à 10 % en poids, mieux encore de 0,01 à 5 % en poids par rapport au poids total de la composition selon l'invention.

De préférence, le ou les agents gélifiants est ou sont contenus dans la phase aqueuse dispersée. Il(s) est ou sont présent(s) en une quantité allant de préférence de 0.01 à 35 % en poids, mieux encore de 0,01 à 25 % en poids par rapport au poids total de la phase dispersée.

La composition selon l'invention comprend de préférence un ou plusieurs polymères filmogènes. Avantageusement, il est contenu dans la phase continue.

Par "polymère filmogène", on entend au sens de la présente demande, un polymère apte à former à lui seul ou en présence d'un agent auxiliaire de filmification, un film continu sur un support, à une température allant de 10 °C à 150 °C.

Le polymère peut être solubilisé ou dispersé sous forme de particules dans la phase continue.

A titre de polymères filmogènes utilisables dans la composition de la présente invention, on peut citer les polymères filmogènes synthétiques obtenus par voie radicalaire ou par polycondensation, les polymères d'origine naturelle et leurs mélanges. D'une manière générale, ces polymères peuvent être des polymères statistiques, des copolymères blocs de type A-B, multi blocs A-B-A ou encore ABCD, ou des polymères greffés.

Par « polymère synthétique obtenu par voie radicalaire » (ou polymère radicalaire), on entend un polymère obtenu par polymérisation de monomères à insaturation(s) notamment éthylénique(s), chaque monomère étant susceptible de s'homopolymériser (à l'inverse des polycondensats).

Les polymères filmogènes radicalaires peuvent être notamment des homopolymères ou des copolymères acryliques et/ou vinyliques.

Les polymères filmogènes vinyliques peuvent résulter de la polymérisation de monomères à insaturation(s) éthylénique(s) ayant au moins un groupement acide, et/ou des esters de ces monomères acides et/ou des amides de ces monomères acides.

Comme monomères à insaturation(s) éthylénique(s) ayant au moins un groupement acide ou monomère porteur de groupement acide, on peut utiliser des acides carboxyliques insaturés α,β-éthyléniques tels que l'acide acrylique, l'acide méthacrylique, l'acide crotonique, l'acide maléique et l'acide itaconique. On utilise de préférence l'acide (méth)acrylique et l'acide crotonique, et plus particulièrement l'acide (méth)acrylique. Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, plus particulièrement en C₁-C₈, des (méth)acrylates d'aryle, en particulier d'aryle en C₆-C₁₀, des (méth)acrylates d'hydroxyalkyle, en particulier d'hydroxyalkyle en C₂-C₆.

Parmi les (méth)acrylates d'alkyle, on peut citer le méthacrylate de méthyle, le méthacrylate d'éthyle, le méthacrylate de butyle, le méthacrylate d'isobutyle, le méthacrylate de 2-éthylhexyle et le méthacrylate de lauryle.

Parmi les (méth)acrylates d'hydroxyalkyle, on peut citer l'acrylate d'hydroxyéthyle, l'acrylate de 2-hydroxypropyle, le méthacrylate d'hydroxyéthyle et le méthacrylate de 2-hydroxypropyle.

Parmi les (méth)acrylates d'aryle, on peut citer l'acrylate de benzyle et l'acrylate de phényle.

De préférence, les esters de l'acide (méth)acrylique sont des (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont remplacés par des atomes de fluor.

Comme amides des monomères acides, on peut citer, par exemple, les (méth)acrylamides, et notamment les N-(alkyl en C₂-C₁₂)(méth)acrylamides. Parmi les N-(alkyl en C₂-C₁₂)(méth)acrylamides, on peut citer le N-éthylacrylamide, le N-t-butylacrylamide et le N-t-octylacrylamide.

Les polymères filmogènes radicalaires vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemples d'ester vinylique, on peut citer l'acétate de vinyle, le néodécanoate de vinyle, le pivalate de vinyle, le benzoate de vinyle et le t-butyl benzoate de vinyle.

Comme monomères styrèniques, on peut notamment citer le styrène et l'alpha-méthylstyrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère vinylique, on peut également utiliser les polymères acryliques siliconés.

On peut également citer les polymères résultant de la polymérisation radicalaire d'un ou plusieurs monomères radicalaires, à l'intérieur et/ou partiellement à la surface de particules préexistantes d'au moins un polymère choisi dans le groupe constitué par les polyuréthanes, les polyurées, les polyesters, les polyesteramides et/ou les résines alkydes. Ces polymères sont généralement appelés « polymères hybrides ».

Comme polymère filmogène obtenu par polycondensation (ou polycondensat filmogène), on peut citer les polyuréthannes anioniques, cationiques, non-ioniques ou amphotères, les polyuréthannes-acryliques, les polyuréthannes-polyvinylpyrrolidones, les polyester-polyuréthannes, les polyéther-polyuréthannes, les polyurées, les polyurée/polyuréthannes, les polyuréthannes siliconés, et leurs mélanges.

Le polyuréthanne filmogène peut être, par exemple, un copolymère polyuréthanne, polyurée/uréthanne, ou polyurée, aliphatique, cycloaliphatique ou aromatique, comportant seule ou en mélange au moins une séquence choisie parmi :
- une séquence d'origine polyester aliphatique et/ou cycloaliphatique et/ou aromatique, et/ou
- une séquence siliconée, ramifiée ou non, par exemple polydiméthylsiloxane ou polyméthylphénylsiloxane, et/ou
- une séquence comportant des groupes fluorés.

Les polyuréthannes filmogènes tels que définis dans l'invention peuvent être également obtenus à partir de polyesters, ramifiés ou non, ou d'alkydes comportant des atomes d'hydrogène mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Parmi les polycondensats filmogènes, on peut également citer les polyesters, les polyesters amides, les polyesters à chaîne grasse, les polyamides, et les résines époxyesters.

Les polyesters peuvent être obtenus, de façon connue, par polycondensation d'acide(s) dicarboxylique(s) avec un ou plusieurs polyols, notamment un ou plusieurs diols.

Le ou les acides dicarboxyliques peut ou peuvent être aliphatique(s), alicyclique(s) ou aromatique(s). On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylgluratique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarbocylique, l'acide 1,4-cyclohexa-nedicarboxylique, l'acide isophtalique, l'acide téréphtalique, l'acide 2,5-norborane-dicarboxylique, l'acide diglycolique, l'acide thiodipropionique, l'acide 2,5-naphtalène-dicarboxylique, l'acide 2,6-naphtalène-dicarboxylique. Ces monomères acide dicarboxylique peuvent être utilisés seuls ou en combinaison d'au moins deux monomères acide dicarboxylique. Parmi ces monomères, on choisit en particulier l'acide phtalique, l'acide isophtalique et l'acide téréphtalique.

Le diol peut être choisi parmi les diols aliphatiques, alicycliques et aromatiques. On utilise en particulier un diol choisi parmi l'éthylène glycol, le diéthylène glycol, le triéthylène glycol, le 1,3-propanediol, le cyclohexane diméthanol, le 1,4-butanediol. Comme autres polyols, on peut utiliser le glycérol, le pentaérythritol, le sorbitol ou le triméthylol propane.

Les polyesters amides peuvent être obtenus de manière analogue aux polyesters, par polycondensation de diacides avec des diamines ou des aminoalcools. Comme diamine, on peut utiliser l'éthylènediamine, l'hexaméthylènediamine, la méta- ou para-phénylènediamine. Comme aminoalcool, on peut utiliser la monoéthanolamine.

On peut aussi utiliser dans la présente invention des polymères d'origine naturelle, éventuellement modifiés, comme la résine shellac, la gomme de sandaraque, les dammars, les élémis, les copals, les polymères cellulosiques insolubles dans l'eau tels que la nitrocellulose et les esters de cellulose modifiés comme des esters de carboxyalkyl cellulose, par exemple ceux décrits dans la demande de brevet US 2003/185774, et leurs mélanges.

De préférence, le polymère filmogène est choisi parmi les polymères cellulosiques insolubles dans l'eau et les esters de cellulose modifiés comme décrits ci-dessus, et leurs mélanges.

Le ou les polymères filmogènes sont présents de préférence en une quantité allant de 10 à 40 % en poids, mieux encore de 15 à 30 % en poids par rapport au poids total de la composition.

La phase continue peut comprendre en outre une ou plusieurs huiles et/ou un ou plusieurs solvants organiques. De préférence, la phase continue comprend au moins un solvant organique et la composition se présente alors sous forme d'émulsion eau-dans-solvant organique.

De préférence, le ou les solvants organiques sont peu solubles ou insolubles dans l'eau.

Par « solvant peu soluble dans l'eau », on entend au sens de la présente invention un solvant dont la solubilité dans l'eau à 20°C et à pression atmosphérique est inférieure à 4%.

Par « solvant insoluble dans l'eau », on entend au sens de la présente invention un solvant dont la solubilité dans l'eau à 20°C et à pression atmosphérique est inférieure à 1% et de préférence inférieure à 0,5%.

A titre d'exemples de solvant organique, on peut notamment citer :
- les cétones liquides à température ambiante telles que la méthylisobutylcétone, la diisobutylcétone ou l'isophorone ;
- les esters à chaîne courte comportant de 5 à 8 atomes de carbone au total, tels que l'acétate de propyle, l'acétate d'isopropyle, l'acétate de n-butyle, l'acétate d'isopentyle ou l'acétate de méthoxypropyle ;
- les éthers liquides à température ambiante tels que le diméthyléther ;
- les alcanes liquides à température ambiante tels que le décane, l'heptane, le dodécane ou le cyclohexane ;
- les aldéhydes liquides à température ambiante tels que le benzaldéhyde ;
- le 3-éthoxypropionate d'éthyle ;
- les carbonates tel que le carbonate de diméthyle ; et
- leurs mélanges.

De préférence, le ou les solvants organiques sont choisis parmi les esters à chaîne courte comportant de 5 à 8 atomes de carbone au total, tels que l'acétate de propyle, l'acétate d'isopropyle, l'acétate de n-butyle, l'acétate d'isopentyle, l'acétate de méthoxypropyle et leurs mélanges.

Le ou les solvants organiques peuvent être présents dans la composition selon l'invention, en une quantité allant de préférence de 5 à 85 % en poids, et en particulier de 25 % à 80 % par rapport au poids total de la composition.

De préférence, la composition de vernis à ongles selon l'invention est exempte d'acétate d'éthyle.

La composition selon l'invention peut comprendre en outre un ou plusieurs agents plastifiants, par exemple dans la phase continue. A titre d'exemples d'agent plastifiant, on peut notamment citer :
- les dérivés d'éthylène glycol tels que les éthers d'éthylène glycol ou de diéthylène glycol, par exemple l'éthyléther de diéthylène glycol, le méthyléther de diéthylène glycol, le butyléther de diéthylène glycol, l'hexyléther de diéthylène glycol, l'éthyléther d'éthylène glycol, le butyléther d'éthylène glycol, ou encore l'hexyléther d'éthylène glycol ;
- les esters de polyol comme le diacétate de propylène glycol ;
- les dérivés de propylène glycol tels que les éthers de propylène glycol ou de dipropylène glycol, et en particulier le phényléther de propylène glycol, le butyléther de dipropylène glycol, le butyléther de tripropylène glycol, le méthyléther de propylène glycol, l'éthyléther de dipropylène glycol, le méthyléther de tripropylène glycol, ou le butyléther de propylène glycol ;
- des esters d'acides, notamment carboxyliques tels que des adipates, des citrates, des esters de l'acide tertio-butylique, des phtalates, des sébaçates, des stéarates, des palmitates, des carbonates, des tartrates, des benzoates, des acétylricinoléates, des glycolates ou des triacétates, et plus particulièrement l'adipate de diisobutyle, l'adipate de diéthyle, le citrate de triéthyle, le citrate de tributyle, l'acétylcitrate de triéthyle, l'acétylcitrate de tributyle, l'acétylcitrate de triéthylhexyle, l'ester de l'acide tertio-butylique et du 2,2,4-triméthylpentane-1,3-diol, le phtalate de diéthyle, le phtalate de dibutyle, le phtalate de dioctyle, le phtalate de dipentyle, le phtalate de diméthoxyéthyle, le phtalate de butyle et de 2-éthylhexyle, le sébaçate de diméthyle, le sébaçate de dibutyle, le stéarate d'éthyle, le palmitate de 2-éthylhexyle, le benzoate de benzyle, l'acétylricinoléate de butyle, l'acétylricinoléate de glycéryle, le glycolate de butyle ou le triacétate de glycéryle ;
- des phosphates, et plus particulièrement le phosphate de tricrésyle, le phosphate de tributyle, le phosphate de triphényle ou le phosphate de tributoxyéthyle ;
- le camphre, le N-éthyl-o,p-toluènesulfonamide ;
- des dérivés oxyéthylénés tels que les huiles oxyéthylénées, notamment les huiles végétales, telles que l'huile de ricin ;
- les huiles siliconées ; et
- leurs mélanges.

Avantageusement, l'agent plastifiant est choisi parmi les esters d'acides tels que décrits ci-dessus et leurs mélanges, et encore plus particulièrement parmi l'adipate de diisobutyle, l'adipate de diéthyle, le citrate de triéthyle, le citrate de tributyle, l'acétylcitrate de triéthyle, l'acétylcitrate de tributyle, l'acétylcitrate de triéthylhexyle, l'ester de l'acide tertio-butylique et du 2,2,4-triméthylpentane-1,3-diol et leurs mélanges.

Le ou les agents plastifiants peuvent être présents dans la composition selon l'invention, en une quantité allant de préférence de 0,1 % à 20 % en poids, et en particulier de 0,5 % à 10 % par rapport au poids total de la composition.

La phase dispersée comprend de préférence majoritairement de l'eau, c'est-à-dire une quantité d'eau allant de préférence de 40 à 99,99 % en poids, mieux encore de 60 à 99,99 % en poids par rapport au poids total de la phase dispersée.

Par rapport au poids total de la composition selon l'invention, l'eau est présente en une quantité allant de préférence de 15 à 90 % en poids, mieux encore de 20 à 70 % en poids.

La composition selon l'invention peut comprendre en outre au moins une matière colorante qui peut être choisie parmi les composés pulvérulents et les colorants hydrosolubles ou liposolubles. La matière colorante peut être présente en une quantité allant de préférence de 0,01 à 50 % en poids, mieux de 0,05 à 30 % en poids et mieux encore de 0,1 à 20 % en poids par rapport au poids total de la composition.

Les composés pulvérulents peuvent être choisis parmi les pigments et/ou les nacres et/ou les paillettes, habituellement utilisés dans les vernis à ongles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium, le cuivre ou le bronze. Parmi les pigments organiques, on peut citer le noir de carbone, les pigments de type D & C, et les laques à base de carmin de cochenille, de baryum, strontium, calcium, aluminium, la guanine.

On peut également citer les pigments à effet tels les particules comportant un substrat organique ou minéral, naturel ou synthétique, par exemple le verre, les résines acrylique, polyester, polyuréthane, poly(téréphtalate d'éthylène), les céramiques, les alumines recouvertes ou non de substances métalliques comme l'aluminium, l'or, le cuivre, le bronze, ou d'oxydes métalliques comme le dioxyde de titane, l'oxyde de fer, l'oxyde de chrome, de pigments minéraux ou organiques et leurs mélanges.

Par « pigments à effet », on entend des pigments conférant une couleur en masse différente de la couleur de reflet, et on peut avoir plusieurs reflets.

Les nacres peuvent être choisis parmi les pigments nacrés blancs, tels que le mica recouvert de titane, ou d'oxychlorure de bismuth, les pigments nacrés colorés, tels que le mica titane avec des oxydes de fer, le mica titane avec notamment du bleu ferrique ou de l'oxyde de chrome, le mica titane avec un pigment organique du type précité ainsi que les fluorophlogopite avec des oxydes de fer.

On peut aussi utiliser des pigments à propriétés goniochromatiques, notamment à cristaux liquides ou multicouches.

Les colorants hydrosolubles sont, par exemple, le jus de betterave et le bleu de méthylène.

A titre d'exemples de colorant liposoluble, on peut notamment citer le rouge Soudan, le DC Red 17, le DC Green 6, le β-carotène, l'huile de soja, le brun Soudan, le DC Yellow 11, le DC Violet 2, le DC orange 5 et le jaune quinoléine.

La composition selon l'invention peut comprendre en outre une ou plusieurs charges, notamment en une teneur allant de 0,01 % à 50 % en poids, de préférence allant de 0,01 % à 30 % en poids par rapport au poids total de la composition. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut comprendre en outre au moins un additif utilisé couramment dans les compositions cosmétiques et connu de l'homme du métier comme étant susceptible d'être incorporé dans une composition de vernis à ongles.

Ces additifs peuvent être choisis parmi les agents de coalescence, les agents épaississants, les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants, les actifs choisis parmi les huiles essentielles, les filtres UV/solaires organiques et minéraux, les parfums, les agents hydratants, les vitamines, les protéines, les céramides , les extraits végétaux, etc.. ; et leurs mélanges.

A titre d'exemples d'agent épaississant, on peut notamment citer les argiles hydrophiles comme les hectorites, les bentonites, comme par exemple la Laponite XLS fournie par la société Rockwood, les agents épaississants associatifs différents des agents gélifiants décrits ci-dessus, tels que les polyuréthanes associatifs comme le Serad FX commercialisé par la société Servo, les agents épaississants cellulosiques hydrosolubles tels que l'hydroxyéthylcellulose, ou les gommes naturelles, telle que la gomme xanthane.

De préférence, la composition de vernis à ongles selon l'invention est exempte d'argile hydrophile. De préférence, la composition de vernis à ongles selon l'invention est exempte de bentonite.

Bien entendu, l'homme du métier veillera à choisir ce ou ces éventuels composés complémentaires et/ou leur quantité, de manière telle que les propriétés de la composition selon l'invention ne soient pas, ou substantiellement pas altérées par l'adjonction envisagée.

La composition telle que définie ci-dessus, sous forme d'émulsion, peut être préparée de la manière suivante :
- en préparant d'abord un gel aqueux par mélange d'eau, un ou plusieurs agents gélifiants et éventuellement d'au moins un actif hydrosoluble ;
- en préparant la phase continue par mélange du ou des solvants organiques et éventuellement d'au moins un autre composé tel que, par exemple, un polymère filmogène, un agent plastifiant ou un colorant ;
- puis en introduisant sous forte agitation le gel aqueux dans la phase continue afin de réaliser l'émulsion.

L'invention concerne également un procédé de maquillage des ongles comprenant l'application sur les ongles d'au moins une couche d'une composition de vernis à ongles telle que définie ci-dessus.

Un autre objet est un article cosmétique comprenant :
i) un récipient comprenant une composition de vernis à ongle telle que définie ci-dessus ; et
ii) un applicateur pour prélever le produit contenu dans le récipient et l'appliquer sur les ongles, l'applicateur étant sous la forme d'un pinceau, d'une spatule, d'un embout en mousse ou d'une lame souple définissant une face applicatrice.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que les polypropylènes (PP) ou les polyéthylènes (PE), ou comme un métal.

Le récipient est équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse ou d'une lame souple définissant une face applicatrice.

L'exemple qui suit illustre de manière non limitative l'invention. Sauf indication contraire, les quantités indiquées sont exprimées en gramme.

### Exemple

On a préparé la composition de vernis à ongles à partir des ingrédients indiqués dans le tableau suivant.

| | |
|---|---|
| Copolymère acrylamide/acrylamido-2-méthylpropane-sulfonate de sodium en émulsion inverse à 40 % en poids dans l'isoparaffine/éthanol⁽¹⁾ | 0,2 |
| Polydiméthylsiloxane (PDMS) oxyéthyléné à terminaisons méthoxy (viscosité : 18-28 cSt) | 0,5 |
| D&C Red 7 | 1,59 |
| D&C Red 6 | 1,59 |
| N-éthyl-o,p-toluènesulfonamide | 4,24 |
| Acétylcitrate de tributyle | 5,30 |
| Eau désionisée | 19,8 |
| Nitrocellulose à 30 % en poids dans l'alcool isopropylique | 20,03 |
| Acétate de butyle qsp | 100 |

| | |
|---|---|
| ⁽¹⁾ vendu sous la dénomination SEPIGEL 305 par la société SEPPIC | |

On prépare tout d'abord le gel aqueux en mélangeant l'eau avec le copolymère acrylamide/acrylamido-2-méthylpropane-sulfonate de sodium.

On prépare ensuite la phase continue en mélangeant l'acétate de butyle, le polymère filmogène, les agents plastifiants, les colorants et le PDMS oxyéthyléné à terminaisons méthoxy. Puis on ajoute sous forte agitation le gel aqueux à la phase continue pour obtenir l'émulsion eau-dans-solvant organique.

La composition de vernis obtenue après mélange des ingrédients ci-dessus, est stable et fluide.

Le vernis s'applique facilement et conduit, après séchage, à un film homogène, brillant, adhérant bien sur l'ongle et flexible.

## Revendications

1. Composition de vernis à ongle sous forme d'émulsion, comprenant au moins une phase continue et au moins une phase dispersée, et un ou plusieurs polymères hydrosolubles ou hydrodispersibles obtenus à partir d'acide acrylamido-2-méthylpropane-sulfonique (ou AMPS) ou d'un de ses sels et de monomères hydrosolubles à insaturation(s) éthylénique(s) comme agent gélifiant.

2. Composition de vernis à ongle selon la revendication 1, **caractérisée en ce que** la phase continue comprend majoritairement une ou plusieurs huiles ou un ou plusieurs solvants organiques, et la phase dispersée est aqueuse.

3. Composition de vernis à ongle selon la revendication 1 ou 2, **caractérisée en ce que** la phase continue de l'émulsion est présente en une quantité allant de 10 à 85 % en poids, mieux de 30 à 80% en poids par rapport au poids total de la composition.

4. Composition de vernis à ongle selon l'une quelconque des revendications 1 à 3, **caractérisée en ce que** la phase dispersée de l'émulsion est présente en une quantité allant de 15 à 90 % en poids, mieux de 20 à 70 % en poids par rapport au poids total de la composition.

5. Composition de vernis à ongle selon l'une quelconque des revendications 1 à 4, **caractérisée en ce que** la phase continue et la phase dispersée de l'émulsion sont présentes en un rapport pondéral allant de 1/10 à 6, mieux de 2/5 à 4.

6. Composition de vernis à ongle selon l'une des revendications 1 à 5, **caractérisée en ce que** l'agent gélifiant est choisi parmi les copolymères obtenus à partir d'AMPS ou d'un de ses sels et d'acrylamide ou de méthylacrylamide, les copolymères d'AMPS ou d'un de ses sels et de vinylpyrrolidone ou de vinylformamide, les copolymères d'AMPS ou d'un de ses sels et d'acrylate de sodium, les copolymères d'AMPS ou d'un de ses sels et d'acrylate d'hydroxyéthyle.

7. Composition de vernis à ongle selon l'une quelconque des revendications précédentes, **caractérisée en ce que** le ou les agents gélifiants sont présents en une quantité allant de 0.01 à 10 % en poids, de préférence de 0.01 à 5 % en poids par rapport au poids total de la composition.

8. Composition de vernis à ongle selon l'une quelconque des revendications 2 à 7, **caractérisée en ce que** le ou les solvants organiques sont choisis parmi les cétones liquides à température ambiante, les esters à chaîne courte comportant de 5 à 8 atomes de carbone au total, les éthers liquides à température ambiante, les alcanes liquides à température ambiante, les aldéhydes liquides à température ambiante, le 3-éthoxypropionate d'éthyle, les carbonates et leurs mélanges.

9. Composition de vernis à ongle selon l'une quelconque des revendications précédentes, **caractérisée en ce qu'**elle comprend un ou plusieurs polymères filmogènes dans la phase continue.

10. Composition de vernis à ongle selon la revendication 9, **caractérisée en ce que** le polymère filmogène est choisi parmi les polymères synthétiques obtenus par voie radicalaire ou par polycondensation, les polymères d'origine naturelle et leurs mélanges.

11. Composition de vernis à ongle selon l'une quelconque des revendications 9 à 10, **caractérisée en ce que** le ou les polymères filmogènes sont présents en une quantité allant de 10 à 40 % en poids, de préférence de 15 à 30 % en poids par rapport au poids total de la composition.

12. Composition de vernis à ongle selon l'une quelconque des revendications précédentes, **caractérisée en ce qu'**elle comprend un ou plusieurs agents plastifiants choisi(s) parmi :
- les dérivés d'éthylène glycol ;
- les esters de polyol ;
- les dérivés de propylène glycol ;
- des esters d'acides ;
- des phosphates ;
- le camphre, le N-éthyl-o,p-toluenesulfonamide ;
- des dérivés oxyéthylénés ;
- les huiles siliconées ; et
- leurs mélanges.

13. Composition de vernis à ongle selon la revendication 12, **caractérisée en ce que** le ou les agents plastifiants sont présents en une quantité allant de 0,1 % à 20 % en poids, et en particulier de 0,5 % à 10 % en poids par rapport au poids total de la composition.

14. Composition de vernis à ongle selon l'une quelconque des revendications précédentes, **caractérisée en ce que** la phase dispersée comprend de 40 à 99,99 % en poids d'eau par rapport au poids total de la phase dispersée.

15. Article cosmétique comprenant :
(i) un récipient, et
(ii) un applicateur pour prélever le produit contenu dans le récipient et l'appliquer sur les ongles, l'applicateur étant sous la forme d'un pinceau, d'une spatule, d'un embout en mousse ou d'une lame souple définissant une face applicatrice, **caractérisé en ce que** le récipient contient une composition de vernis à ongle selon l'une quelconque des revendications 1 à 14.
